## Europäisches Patentamt

## European Patent Office

## Office européen des brevets

Publication number: **0 298 656**

**A1**

## EUROPEAN PATENT APPLICATION

21 Application number: **88305943.8**

22 Date of filing: **30.06.88**

51 Int. Cl.⁴ **C12Q 1/68**

30 Priority: **10.07.87 GB 8716367**

43 Date of publication of application:
**11.01.89 Bulletin 89/02**

84 Designated Contracting States:
**BE CH DE ES FR IT LI NL SE**

71 Applicant: **Reeders, Stephen Thomas**
**2073 LMP, Nephrology Yale University**
**School of Medicine 333 Cedar Street**
**New Haven, CT 06510(US)**

72 Inventor: **Reeders, Stephen Thomas**
**2073 LMP, Nephrology Yale University**
**School of Medicine 333 Cedar Street**
**New Haven, CT 06510(US)**

74 Representative: **Halstead, John Miles**
**54 Pine Walk**
**Carshalton Beeches, Surrey SM5 4HD(GB)**

54 Polynucleotide probes.

57 The invention provides compositions and methods for improved identification of individuals and individual members of species, by use of regions of DNA hypervariability found in all cells, otherwise called hypervariable regions, of HVRs, in which the DNa contains tandem repeats. The number of repeats varies considerably from the individual to another. Many such regions of the human genome can be probed simultaneously to display this variability using any DNA or other polynucleotide probe of which the essential constituent is a short core sequence, tandemly repeated at least 3 times. Probing with each of the probes reveals differences in genomic DNA at multiple highly-polymorphic hypervariable regions to produce an individual-specific DNA 'fingerprint' of general use for genetic identification purposes, paternity and maternity testing, testing of the relatedness of individuals and the relationship of one individual to another (i.e. family structure analysis), forensic medicine, the diagnosis of genetic diseases and cancer, assessment of bone marrow engraftment, study of pedigrees of non-human species, and identification of individual members of these species. Probing can be carried out on DNA from any cellular tissue.

FIG.2

## Polynucleotide Probes

### Background of the Invention

#### (1) Field of the Invention

The invention relates to polynucleotide compositions which can be labelled to serve as probes and used in examining the human or animal genome, and to methods of identifying genomic DNA using such probes. The methods of identification are useful, for example, in paternity and maternity testing, forensic medicine and in the diagnosis of genetic diseases and cancer.

#### (2) Description of the prior art

A main prior method of identifying genetic variation is carried out by studying blood group or protein polymorphisms, tissue antigens, or restriction fragment length polymorphisms (RFLPs) in genomic DNA. The identification of the locus of the DNA defect responsible for adult polycystic kidney disease, by S.T. Reeders et al., Nature 317, 542-544, (1985), and the analysis of predisposition to retinoblastoma by W.K. Cavanee et al., Nature 305, 779-784, (1983) are examples of the use of DNA polymorphisms to detect genetic variations associated with genetic diseases. Reference is made to British Patent Application 2,166, 445 A of A.J. Jeffries, published 8 May 1986.

Most RFLPs result from small scale changes in DNA, usually base substitutions, which create or destroy specific restriction endonuclease cleavage sites. Since the mean heterozygosity of human DNA is low (approximately 0.001 per base pair), restriction endonucleases will seldom detect a RFLP at a given locus. Even when detected, most RFLPs are only dimorphic (presence and absence of a restriction endonuclease cleavage site) with a heteroxygosity, determined by allele frequencies, which does not exceed 50% and which is usually much less. As a result, all such RFLPs will be uniformative in pedigree analysis whenever critical individuals are homozygous. Pedigree analysis and the identification of individuals using blood groups etc. are also limited by their relatively low informativeness, or low information content.

Genetic analysis is considerably simplified by the availability of probes for hypervariable regions (HVRs) of DNA which show multiallelic variation and correspondingly high heterozygosities. The first such region was isolated by A.R. Wyman et al., Proc. Natl. Acad. Sci USA 77, 6754-6758 (1980), by chance from a library of random segments of human DNA. The structural basis for multiallelic variation at this locus is not yet known. Subsequently, and again by chance, several other highly variable regions (HVRs) have been discovered near the human insulin gene (G.I. Bell et al., Nature 295; 31-35 (1982)), the c-Ha-ras-1 oncogene (D.J. Capon et al., Nature 302, 33-37 (1983)), and the myoglobin gene (P. Weller et al., EMBO J, 3, 439-446 (1984). In each case, the variable region consists of tandem repeats of a short sequence (a core sequence) and polymorphism is due to allelic differences in the number of repeats. The resulting length variation of the HVR can be detected using any restriction endonuclease which cleaves frequently but does not cleave the repeat unit.

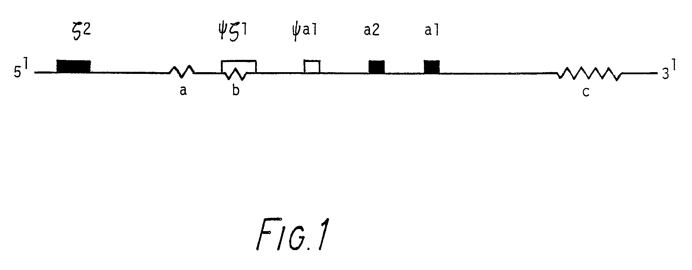

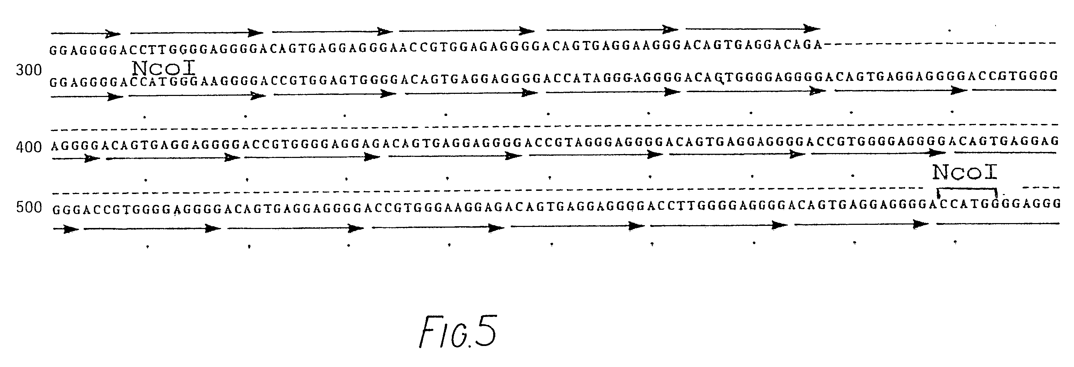

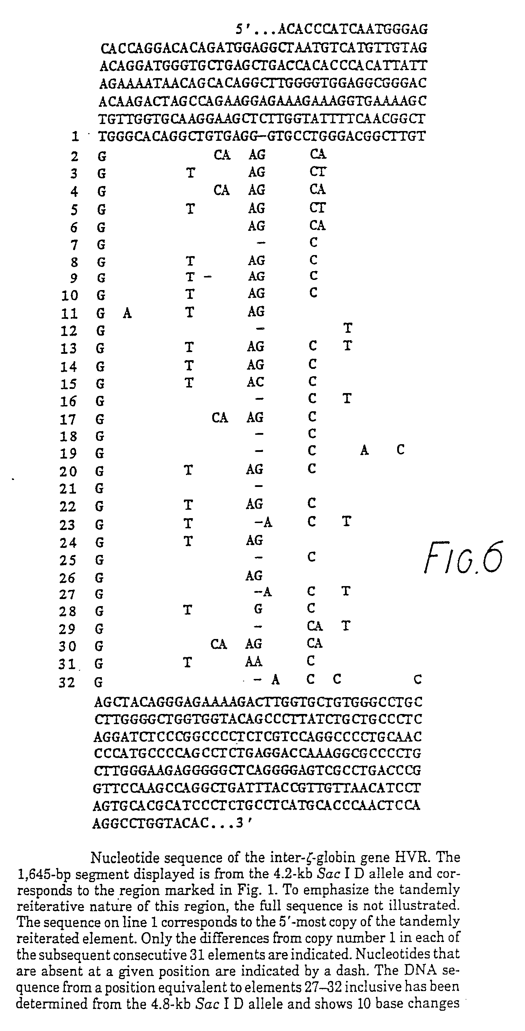

There have previously been described three related hypervariable regions (HVRs) from the region of the alpha-globin cluster (Higgs DR et al, Nucleic Acids Research (1981), vol 9, p4213-4224; Reeders ST et al, Nature (1985) vol 317, p542-544; Nicholls et al, Nucleic Acids Research (1985) vol 13, p7569-7578; Goodbourn SEY et al, Proc Natl. Acad. Sci. USA (1983) vol 80, p5022-5026; Jarman AP et al, EMBO Journal (1986) vol 5, p1857-1863; Goodbourn SEY et al Mol. Biol. Med. (1984) vol 2, p223-238). The map of these HVRs is shown in figure 1. Each of these HVRs (the 3′ HVR, interzeta HVR, and zeta HVR) consists of any array of tandemly-repeated units. They are related in that the repeat units contain a consensus core sequence, that is a sequence which can be identified as a substantial or perfect match between the repeat units of two or more hypervariable regions (HVRs) from different loci (figures 5,6,7). This consensus sequence of the three HVRs is also shared by a fourth HVR, the insulin HVR (Jarman et al, EMBO Journal (1986) vol 5, p1857-1863; Bell GI et al, Nature (1982) vol 295, p31-35). These particular four probes illustrate the present invention. When polynucleotide probes made from these HVRs are radiolabelled and hybridised to genomic DNA under high-stringency conditions (e.g. see Reeders ST., Nature (1985), vol 317, p542-544), each recognises a locus-specific polymorphism. Under these conditions only the 3′ HVR and insulin HVR are highly polymorphic. The sequence relationships between these four HVRs, to which this invention particularly relates, suggested that they might belong to a larger family of HVRs, all containing tandem repeats with core sequences having substantial homology to one another, so that any one of the probes might hybridise to a subset of the members of the family in genomic DNA if the correct hybridisation conditions could be determined. A first object of the invention is to provide a way to probe genomic DNA using cloned fragments containing any one of these related HVRs in such a way as to reveal variability simultaneously in many HVRs thereby generating a pattern of hybridised fragments which is termed a DNA "fingerprint". The present invention is based on the discovery that many highly polymorphic HVRs in both human DNA and animal DNA of many species contain a region of DNA sequence which is homologous with one of these particular (3'HVR, interzeta HVR, zeta HVR, insulin HVR).

A second object of the invention is to permit each of the HVR probes to be used alone or preferably with other HVRs to detect multiple highly polymorphic HVRs in a certain set of animal species; each probe used alone is not necessarily sufficient to generate a DNA fingerprint in any given species. It is reasoned that, during the evolutionary divergence of the species, mutations have occurred in some HVRs but not in others so that any particular HVR may or may not have sufficient homology to any one of the four human HVRs of the invention to permit DNA fingerprinting with a DNA probe containing that particular HVR. However, at least one of the four probes can be used under appropriate conditions for the purpose of identifying an individual animal from any one of the species that has been examined to date (see example 3).

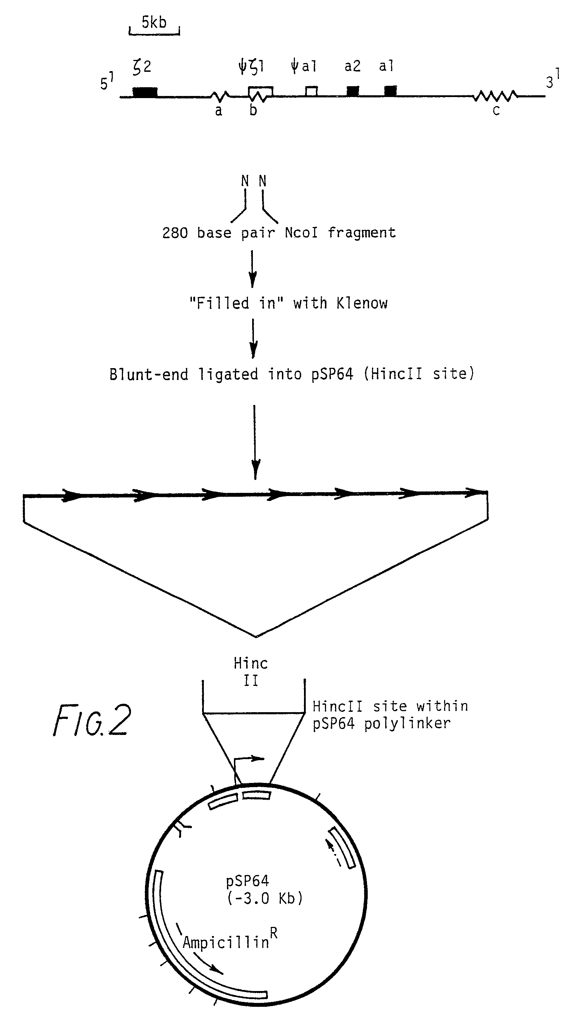

A third object of the invention is to permit construction of a series of plasmids with inserts containing each of the four HVRs cloned into pSP64/65 vectors (figures 2,3,4). These constructs permit the preparation of radiolabelled RNA probes of high specific activity using the SP6 method. These highly sensitive probes allow a DNA fingerprint to be generated after much shorter periods of autoradiography than can be achieved by other means of probing. This method of labelling also permits the detection of an increased number of related HVRs some of which, because of a relatively low degree of homolgy with the core sequences of the probes, are weakly hybridising. These plasmid constructs, when labelled by the SP6 method, also permit the identification of individuals for smaller amounts of DNA than must be used with other labelling methods. Consistent results have been obtained from $0.2\mu g$ genomic DNA.

The core component of the probes can be defined in various ways founded on the same underlying principles. The most fundamental underlying principle is that the repeat sequence of the probe shall consist of or include a nucleotide sequence from a common core region, common to HVRs of human or animal genomic DNA. The common core region is 'common' in the sense of displaying a high degree of consensus, e.g. at least 80%, as between one HVR and another. These HVR's are detectable e.g. by probing genomic DNA fragments with the 3'HVR repeat sequence, to yield hybridised fragments herein referred to as 3'HVR positive fragments. These fragments and the 17 bp repeat of the 3'HVR contain an approximately 11 bp common core sequence. The 3'HVR positive fragments can themselves be used as probes of genomic DNA to generate further fragments which also have the common core sequence.

Another principle is that the core nucleotide sequence shall be not so short that it fails to hybridise effectively to the HVRs of the sample DNA, nor so long that it fails to detect the polymorphisms well. Generally, the core should have from 6 nucleotides up to the maximum found in the common core of minisatellites, approximately 11. The repeat sequence of the probe need not consist entirely of the core but can contain a small number of flanking nucleotides on either side of the core sequence. The repeating units need not be exact repeats either as to number or kind of nucleotides and either as to the core or non-core components of the repeating units. It is, however, convenient to describe and define them herein as repeating units, notwithstanding that this is an approximate term. The block of $\underline{n}$ repeating units can be flanked on either side by any nucleotide sequence, the extent and kind of which is ordinarily irrelevant.

Polynucleotides of the invention include specifically those defined in each of the following ways:

## First definition

Polynucleotides having the general formula, read in the 5' →3' sense

H.(J.core.K)$_n$.L

wherein:

'core' represents a sequence

GXGGGGYACAG

where X is A or C or T or G

and Y is A or C or T or G

or Nucleotide Y may be absent

J and K together represent 0 to 25 additional nucleotides within the repeating unit; and

H and L each represent 0 or at least 1 additional nucleotide flanking the repeating units, and provided that:

    i) 'core' and J and K do not necessarily have the same sequence or length in each (J.core.K) repeating unit;

    ii) 'core' can also represent a variant core sequence;

iii) total actual core sequences in all n repeating units have at least 70% homology with total 'true' core sequences as defined above.

The invention further includes polynucleotides having the general formula
H.(J.core.K.)$_n$.L
wherein:

'core' represents a sequence of from 6 to 16 consecutive nucleotides, read in the same $5' \rightarrow 3'$ sense, selected from (1) the $5' \rightarrow 3'$ common core region of a first human or animal HVR obtained by probing human or animal genomic DNA with the $3'$ HVR interzeta HVR, zeta HVR or insulin HVR, (2) the $5' \rightarrow 3'$ common core region of a second human or animal HVR ob tained by probing human or animal DNA with a probe DNA containing a tandem repeat sequence comprising the common core region of the first HVR, and (3) and $5' \rightarrow 3'$ common core region of a third human or animal minisatellite obtained by probing human or animal genomic DNA with a probe DNA containing a tandem repeat sequence comprising the common core region of the second HVR each said tandem repeat sequence being a repeat of at least 3 units, and polynucleotides of complementary sequence to the above.

The invention also includes polynucleotides of DNA, RNA and of any other kind hybridisable to DNA. The polynucleotides as defined above are unlabelled and can be in double stranded (ds) or single stranded (ss) form.

The invention includes labelled polynucleotides in ss-form for use as probes as well as their labelled ds-precursors, from which the ss-probes can be produced.

They are preferably labelled by the SP6 method but can alter natively be radiolabelled by other means well known in the hybridisation art.

The invention also includes a method of identifying a sample of human or animal genomic DNA which comprises probing said DNA with a probe of the invention and detecting hybridised fragments of the DNA.

This aspect of the invention may involve:
fragmenting total DNA from a sample of cellular material using a restriction endonuclease,
hybridising highly variable DNA fragments with a probe as defined above which contains, in addition to a labelled or marker component, a repeated core component, and
determining the label or marker concentration bound to DNA fragments of different length, or more generally to bands of different molecular size.

Normally, the fragmented DNA is sorted or segregated according to chain length, e.g. by electrophoresis, before hybridisation, and the marker concentration is sensed to obtain a characteristic pattern, individual elements of which are of specific genetic origin.

## SUMMARY OF THE INVENTION

According to a first aspect of the present invention there is provided a polynucleotide in isolated or cloned form having the formula. read in the $5' \rightarrow 3'$ sense
H.(J.core.K)$_n$.L
wherein:

(a) 'core' represents a sequence having at least 6 consecutive nucleotides, selected from the following sequences:
GXGGGGYACAG
and wherein:

(b) (i) X is A or C or T or G
and Y is A or C or T or G
or nucleotide Y may be absent;
(ii) J and K together represent 0 to 25 addition nucleotides within the repeating unit; and
(iii) H and L each represent 0 or at least 1 additional nucleotide flanking the repeating units;
(iv) the repeating unit (J.core.K) need not be an exact repeat either as to number and kind of nucleotides, provided that the polynucleotide has a recognisably repeated core sequence;
(v) 'core' can also represent a variant core sequence, provided that actual core sequences in all n repeating units have an average at least 70% homology with true 'core' sequences as defined above with respect to formula (2)
or a polynucleotide of complementary sequence to the above,
the polynucleotide being capable of hybridising with fragments of DNA from more than one hypervariable region generated by one or more restriction enzymes which do not cleave within a sequence corresponding to the repeating unit (J.core.K).

According to a second aspect of the present invention there is provided a polynucleotide in isolated or cloned form having the general formula, read in the $5' \rightarrow 3'$ sense
H.(J.core.K)$_n$.L
wherein H, J, K, L and 'n' are as defined in claim 1 and:
'core' represents a sequence of from 6 to 16 consecutive nucleotides, read in the same sense, selected from

(1) the common core region of a first animal or human hypervariable region obtained by probing animal or human genomic DNA with a probe containing a $3'$ alpha-globin, or zeta-intron, or interzeta tandem repeat sequence

(2) the common core region of a second animal or human hypervariable region obtained by probing animal or human genomic DNA with a probe containing a tandem repeat sequence comprising (1)

(3) the common core region of a third animal or human hypervariable region obtained by probing animal or human genomic DNA with a probe containing a tandem repeat sequence comprising (2)

each said tandem repeat sequence being a repeat of at least 3 units, or a polynucleotide complementary to the above,

the polynucleotide being capable of hybridising with fragments of DNA from more than one hypervariable region produced by one or more restriction enzymes which do not cleave within a sequence corresponding to the repeating unit (J.core.K).

According to a third aspect of the present invention there is provided a polynucleotide in isolated or cloned form having the general formula, read in the $5' \rightarrow 3'$ sense

H.(J.core.K)$_n$.L

wherein:

'core' represents any of the sequences having at least 6 consecutive nucleotides from within a common core region of a plurality of hypervariable regions of human or animal genomic DNA which displays at least 75% consensus;

'core' does not necessarily have the same sequence in each repeating unit and all other symbols are as defined in claim 1,

or a polynucleotide of complementary sequence to the above, the polynucleotide being capable of hybridising with fragments of DNA from more than one hypervariable region generated by one or more restriction enzymes which do not cleave within a sequence corresponding to the repeating unit (J.core.K).

The invention further provides a method of preparing a polynucleotide having polymorphic hypervariable region-length-specific binding characteristics comprising

(i) identifying a natural tandem repeat sequence in DNA which is capable of limited hybridisation to other polymorphic DNA regions,

(ii) identifying a natural consensus core sequence of the repeat sequence putatively responsible for such binding, and

(iii) isolating or artificially binding a perfect or imperfect tandem repeat sequence derived from the natural consensus core sequence having hypervariable region binding properties which exhibits lower genome locus specificity and higher polymorphic fragment acceptance than the natural repeat sequence.

There is also provided a polynucleotide probe comprising, with the inclusion of a labelled or marker component, a polynucleotide comprising at least three tandem repeats (not necessarily exact repeats) of sequences which are homologous with a hypervariable region of the human or animal genome to a degree enabling hybridisation of the probe to a corresponding DNA fragment obtained by fragmenting the sample DNA with a restriction endonuclease, wherein:

(a) the repeats each contain a core which has at least 75% homologous with a consensus core region of similar length present in a plurality of hypervariable regions from different genomic loci;

(b) the core is at least 6 nucleotides long;

(c) the total number of nucleotides within the repeating unit which do not contribute to the core is not more than 25;

(d) the probe is capable of differentiating DNA by reference to more than one polymorphic hypervariable region.

According to a further feature of the invention there is provided a method of characterising a test sample of genomic DNA, by reference to one or more controls, which comprises probing the test sample with a polynucleotide probe comprising (with the inclusion of necessary of a labelled or marker component), a polynucleotide comprising at least three tandem repeats (not necessarily exact repeats) of sequences which are homologous with a hypervariable region of a genome to a degree enabling hybridisation of the probe therewith, detecting hybridising fragments of DNA, and comparing the hybridised fragments with a said control or controls, wherein:

(a) the sample DNA is fragmented with one or more restriction enzymes which do not cleave to any relevant extent a sequence corresponding to the said tandem repeat;

(b) the repeats each contain a core which is at least 75% homologous with a consensus core region of similar length present in a plurality of hypervariable regions from different genomic loci;

(c) the core is at least 6 nucleotides long;

(d) the total number of nucleotides within the repeating unit which do not contribute to the core is not more than 25;

(e) the hybridised fragments differentiate the sample DNA by reference to more than one polymorphic hypervariable region.

Definitions

The following definitions used in the present invention and the above-mentioned prior art may be of assistance.

## Hypervariable region (HVR)

A region of human or animal DNA at a recognised locus or site is said to be hypervariable if it occurs in many different forms e.g. as to length or sequence.

## Restriction fragment length polymorphism (RPLF)

Is genetic variation in the pattern of human or animal DNA fragments separated after electrophoresis and detected by a probe.

## Minisatellite

A region of human or animal DNA is comprised of tandem repeats of a short DNA sequence. All repeat units may not necessarily show perfect identity. (Probes of the invention comprise minisatellites which are polymorphic).

## Polymorphic

A gene or other segment of DNA which shows variability from individual to individual is said to be polymorphic.

## Core (sequence)

Originally used in the sense of consensus core sequence, but extended to any repeated or variant sequence derived therefrom.

## Consensus core (sequence)

A sequence which can be identified as a substantial or perfect match between the repeat units of two or more minisatellites of differing origin or loci.

## Repeat (sequence)

A sequence which is a perfect or imperfect tandem repeat of a given core sequence or segment containing the core sequence.

## Defined core (sequence)

A core sequence fully consistent with one of formulae (2) to (8) within its own length.

## Variant (core sequence)

An actual core sequence which differs from a defined core sequence to a minor extent (> 50% homology).

## Perfect repeat (sequence)

A sequence which is an exact tandem of a given core sequence or of a segment containing the core sequence.

## Imperfect repeat (sequence)

A sequence in which at least one unit differs in base pair substitution and/or length from at least one other unit. (There will normally be at least three tandem repeats in a probe sequence within which there will normally be at least one defined core sequence and at least one variant).

## % Homology

In comparing two sequences of the same length, the number of base pairs (bp) less the number of bp substitutions in one necessary to give the other, as a percentage of the number of bps.

Nucleotide (nt) and base pair (bp) are used synonymously. Both can refer to DNA or RNA. the abbreviations C, A, G, T refer conventionally to (deoxy)cytidine, (deoxy)adenosine, (deoxy)-guanosine and either deoxythymidine or uridine.

The tandem repeat sequence (artificial or a natural isolate) may thus be a perfect repeat but is preferably an imperfect repeat.

Production of a probe may involve isolation of a natural HVR by cloning and identification by DNA sequencing. It may also involve excision of the required core and its subsequent conversion into a tandem repeat, or the stimulation of unequal exchanges with core fragments of different origin. It may also include cloning of the polynucleotide.

On the other hand, the building step may include synthesising the identified consensus core sequence or a fragment thereof. The consensus core sequence preferably contains not less than 6 bp and preferably contains not more than 16 bp. A tandem repeat of the synthetic core is then constructed.

Naturally the polynucleotide may be the result of a succession of operations at different times following cloning of successful or partially successful intermediates and may include fragments of natural or synthetic origin, so that the end polynucleotide may bear little resemblence to the parent minisatellite.

The probes of the invention are useful in the following areas:

(1) Paternity and maternity testing in man.

(2) Family group verification in e.g. immigration disputes and ihneritance disputes.

(3) Zygosity testing in twins.

(4) Tests for inbreeding in man.

(5) General pedigree analysis in man.

(6) Identification of loci of genetic disease in man, thereby enabling specific probes to the constructed to detect a genetic defect.

(7) Forensic medicine (a) fingerprinting semen samples from rape victims, (b) fingerprinting blood, hair and semen samples from e.g. soiled clothing, (c) identification of human remains.

(8) Cell Chimaerism studies, e.g. following donor versus recipient cells after bone marrow transplantation.

(9) Livestock breeding and pedigree analysis/authentication. (This could include, for example, the routine control and checking of pure strains of animals, and checking pedigrees in the case of litigations involving e.g. race horse and dog breeding). Also to provide genetic markers which might show association with inherited traits of economic importance.

(10) Routine quality control of cultured animal cell lines, checking for contamination of pure cell lines and for routine identification work.

(11) Analysis of tumour cells and tumours for molecular abnormalities.

(12) Polynucleotides or probes derived therefrom have a use in plant breeding.

EXAMPLE: 1 THE DETECTION OF MULTIPLE HIGHLY POLYMORPHIC 'HYPERVARIABLE' GENOMIC LOCI USING A PROBE CONTAINING THE 3' HYPERVARIABLE REGION OF THE ALPHA GLOBIN CLUSTER

## Preparation of the 3' HVR probe

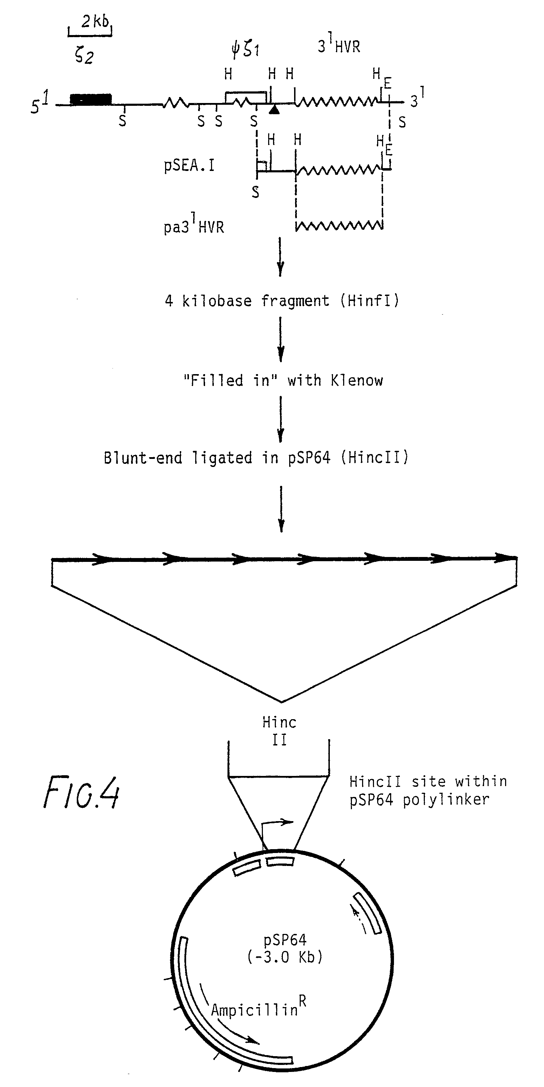

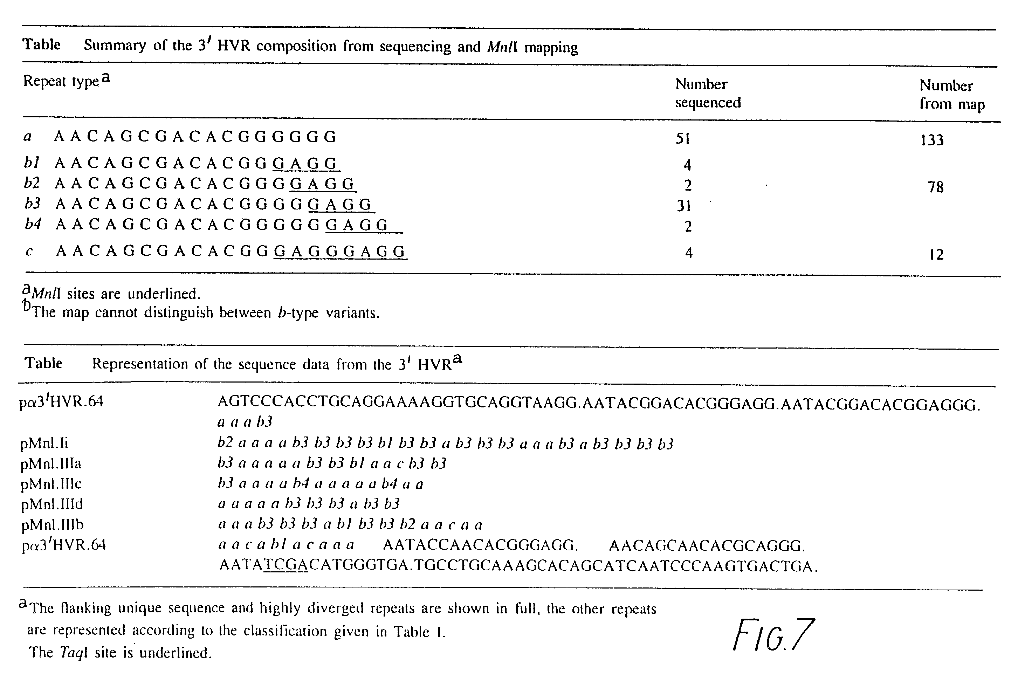

The 3'HVR was isolated from a library of human genomic DNA fragments cloned into the plasmids pSP64 and pSP65 by the methods of Nicholls et al (Nucleic Acids Research (1985) vol 13, p7569-7578) and Jarman et al (EMBO Journal (1986) vol 5, p1857-1863). The probes pSEA1 and palpha 3'HVR.64 can both be used in the methods described below. These constructs are illustrated in figures 4 and 7.

## Radiolabelling of the 3'HVR probe

The 3'HVR probes were labelled by the PS6 method of Melton DM et al (Nucleic Acids Research (1984) vol 12, p7035-7056), with modifications as described in "In vitro RNA synthesis", Amersham International plc, Amersham, UK. Care must be taken to use RNase-free solutions and components. Diethyl pyrocarbonate (DEPC) treated water can be used to protect solutions from RNase. SP6 labelling was carried out using the Amersham SP6 labelling kit with $^{32}$P-UTP. After SP6 polymerase directed transcription of the DNA template, DNA was digested away from the RNA transcript by RNase-free DNase as described in Melton et al (see above). Unincorporated rNTPs were removed by passing the reaction mixture through a G50-80 column. DNase was removed by phenol-chloroform extraction and precipitated with ammonium acetate and ethanol as described in the Amersham protocol. The probe was redissolved in 100 microliters TE and an aliquot taken for estimation of $^{32}$P incorporation by standard methods. Approx. one-tenth of the total labelled probe is sufficient for preparation of 15ml of hybridisation solution.

Other standard labelling methods can also be used to label the 3'HVR probe for the purposes described in the examples but these methods (eg. nick translation, random oligonucleotide priming) have been found to be less sensitive and to require longer periods of autoradiography to detect weakly hybridising fragments.

## Hybridisation, filter washing and autoradiography

Several alternative hybridisation protocols have been found to give satisfactory results. By one such method filters were prehybridised for 15 min. and hybridised for at least 4h. in Church and Gilbert buffer at 65 degC. In a second method, up to 3 filters were prehybridised for at least 3h. at 37degC in 15ml of prehybridisation buffer (50ml of buffer was made by adding 7.5ml 20xSSc, 25ml formamide, 50ml heparin (50mg/ml), 1ml 10%SDS, 16.5ml water). Filters were hybridised overnight at 37degC in 7.5ml of buffer made as follows: 250ml buffer consists of 7.5ml 20xSSC, 25ml formamide, 10ml 25% dextran sulphate, 200 ml heparin (50mg/ml), 1ml 10%SDS, 6.5ml water. Filters were washed at 37degC in 1xSSC, 0.1%SDS for an hour with four changes of prewarmed washing solution.

Autoradiography was carried out using Kodak XAR5 film (not preflashed) without intensifying screens. Exposures of from 3 - 72 hours at 80degC were required to detect all hybridising HVRs. The duration of autoradiography depended on the amount of DNA on the filter and other factors well-known in the hybridisation art.

## Fingerprinting human genomic DNA

DNA may be prepared from leucocytes by any standard method. 1μg of DNA from a number of unrelated individuals was digested with a number of restriction enzymes having a 4bp recognition sequence (eg MboI, HaeIII, HinfI) and electrophoresed for 44h. on a horizontal 0.8% agarose gel in acetate buffer and blotted by Southern's method. Different fingerprints are obtained with different enzymes, but the number of detectable bands is not, on average affected by the choice of enzyme. Conditions of electrophoresis were such that a 2 kilobase λHindIII marker migrated the length of the gel which was either 20cm or 30cm. Nylon filters (Amersham Hybond N) were used routinely but equally good results were obtained with nitrocellulose filters. DNA was cross-linked to membranes according to the manufacturers recommendations.

The results obtained using plasmid palpha3'HVR.64 to hybridise HinfI digested genomic DNA according to the above protocol are shown in fig 1. Fragments of 2.5-23kb were resolved. The pattern of fragments was analysed in over 100 individuals and was extremely polymorphic such that the hybridisation provile provided an individual-specific DNA fingerprint. Each DNA fingerprint was compared with the pattern in the adjacent gel track and the samples were scored for the presence or absence of any band which was present in either sample. The intensity of each band was also taken into consideration when comparing tracks. The frequency of band-sharing was found to depend on the size of the band, being less for large fragments than small ones. On average 14 bands could be resolved or 'scored' in each fingerprint (56 individuals examined). The probability that a band of a certain size and hybridisation intensity present in one sample was also present in an unrelated sample was 0.16 (average figure for bands of all sizes). The probability that two unrelated individuals have indistinguishable fingerprints was estimated to be $10^{-10}$, based on band-sharing frequency, the average heterozygosity, and the number of allelic fragments estimated from pedigree studies (see below). These estimates are based on the degree of resolution obtained in six consecutive gels containing 56 unrelated samples treated according to the methods described above.

Serial dilution of samples of DNA from unrelated individuals were carried out to determine the sensitivity of the 3'HVR probe when labelled by the SP6 method. After 72h of autoradiography, all but one of the bands observed in a fingerprint of 5μg DNA could be observed in a 0.2μg. sample. The high sensitivity of this method of DNA fingerprinting is of importance in forensic medicine where the DNA available for analysis may be limited.

EXAMPLE 2: THE DETECTION OF MULTIPLE HIGHLY-POLYMORPHIC HYPERVARIABLE GENOMIC LOCI USING PROBES CONTAINING THE INTERZETA HVR AND THE ZETA HVR. THE COMBINED USE OF THESE PROBES WITH THE 3'HVR PROBE.

## Preparation of the interzeta HVR probe

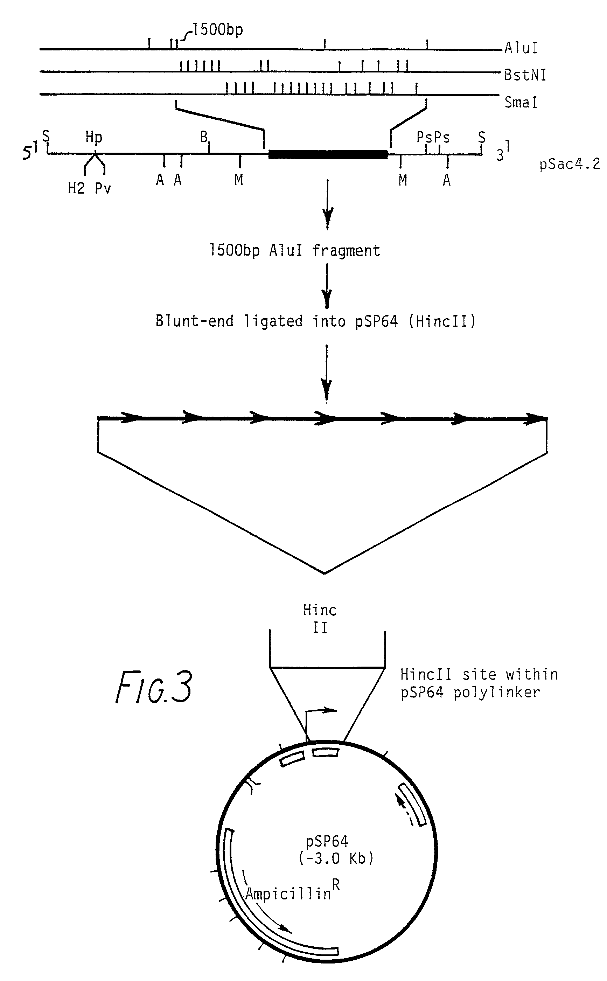

The interzeta probe was prepared by the method of Goodbourn et al (Proc. Natl. Acad. Sci USA (1983) vol 80, p5022-5026). The 1.4kb AluI fragment containing a series of direct repeats was subcloned into pSP64 to permit labelling by the SP6 method (figure 3).

## Preparation of the zeta HVR probe

The zeta HVR was prepared by the method of Proudfoot et al (Cell (1982) vol 31, p553-563). A 280bp NcoI fragment containing direct repeat sequences from intron I of the pseudozeta gene was subcloned into pSP64 (figure 2).

The interzeta and zeta HVRs were used in the same way as described for the 3'HVR in example 1. Both probes gave an extremely polymorphic pattern of bands when hybridised to Southern blots containing digested genomic DNA prepared as in Example 1, and both provided DNA fingerprints of use in the identification of individuals. On average 12 bands could be clearly resolved in each interzeta HVR fingerprint and 15 bands in each zeta HVR fingerprint from HinfI digested human leucocyte DNA.

Nylon filters were sequentially hybridised with the 3'HVR, interzeta HVR and zeta HVR in turn to give 3 different fingerprints of the same sample. Probe DNA was removed after each use by the method described in the filter manufacturers produce sheet. The probability that two unrelated individuals have the same fingerprints for all 3 probes was estimated to be less than $10^{-25}$ after

allowing for the fact that some hypervariable loci may be recognised by all 3 probes.

To examine the stability of the DNA fingerprints obtained with the three probes of the invention, leucocyte DNA was compared with skin DNA, sperm DNA, and DNA from EBV-transformed lymphocytes. All 3 sources of DNA gave the same fingerprint. DNA from various tumours (colonic carcinoma, breast carcinoma, lymphoma) gave fingerprints which are different (in respect of the loss or gain of bands) from DNA from normal tissue and leucocytes from the same individual. This demonstrates that these probes are capable of detecting somatic mutations in tumour tissue.

Pedigree studies were carried out to test the inheritance of hybridising fragments detected by the HVRs of the invention. Mendelian inheritance was observed for each scored fragment. The heterozygosity of fragments of >10kb was estimated as >97%. Infrequently a fragment which was observed in a child was not observed in either parent. The frequency of these 'new' mutant bands was estimated as 1 per 800 resolvable bands. The occurrence of somatic or germline mutations must be taken into account when fingerprinting for the purposes of the verification of family structure e.g. paternity testing.

## EXAMPLE 3: DNA FINGERPRINTING ANIMALS

DNA samples were prepared from leucocytes from horses, cats, dogs, sheep, mice, rats, cattle, chinese hamsters, Russel's viper, pit viper, sparrow, chicken, duck and frog. Samples were digested with either MboI, HinfI, or HaeIII. DNA fingerprints were obtained for all these animals with at least one of the three probes of the invention. Satisfactory fingerprints were not obtained for all combinations of probe and species. For instance, only 5 hybridising fragments were observed using the interzeta probe on horse DNA, whereas a highly-polymorphic pattern with 13 scorable fragments was observed with the zeta HVR. The frequency of band-sharing with this probe was less than 0.2, so that individual-specific fingerprints were obtained which were suitable for use in identification of individual horses. The results for the horse are better than those obtained by using the minisatellite probes λ 33.6 and λ33.15 (by the methods outlined in British Patent Application 2,166, 445 A, published 8 May 1986) in that the number of detectable bands is greater and the band-sharing frequency is less. In view of the considerable degree of inbreeding in thoroughbred horse lineages, the probes of this invention offer a particular advantage in identifying these horses and in verifying pedigree relationships.

## EXAMPLE 4: GENETIC LINKAGE ANALYSIS

The study of the hereditary component of diseases has been accelerated by the use of DNA polymorphisms as linkage markers. However, this approach has been limited by the low heteryzogisity of many marker loci, and the effort and expense of examining loci individually using locus-specific probes. In addition, many different enzymes digests are typically required to detect a reasonable number of RFLPs which also increases the effort, expense, duration of the study, and the consumption of valuable patient DNA. The use of the HVR probes described here permits the analysis of many loci using a single enzyme digest and only 4 probes. Allowing for the fact that four pairs of loci detected with these 4 probes are closely linked, it is estimated that at least 50 loci can be examined for genetic linkage with a disease phenotype if samples from at-risk members of an affected pedigree are examined by the methods described here.

## IN THE DRAWINGS:

Figure 1 illustrates the alpha-globin cluster showing the alpha and zeta genes and (a) the interzeta HVR, (b) the zeta HVR and (c) the 3′ HVR;

Figure 2 shows the steps involved in a method for subcloning the zeta HVR into the plasmid vector pSP64, and wherein NcoI digestion of the pBRs plasmid (Proudfoot et al, Cell (1982), vol 31, p553-563) yields a 280bp NcoI fragment which was blunt ended with Klenow fragment and ligated into the HincII site in the polylinker of pSP64;

Figure 3 shows the steps involved in a method for subcloning the interzeta HVR into pSP64, and wherein AluI digestion of pSAC4.2 (Goodbourn et al, Proc. Natl, Acad. Sci USA (1983) vol 80, p5022-5026) yields a 1500bp fragment which was litgated into the HincII site of the pSP64 polylinker;

Figure 4 is an illustration of preparation of the pSEA1 and pa3'HVR clones containing the tandem-repetitive 3′ HVR;

Figure 5 shows the 5′ → 3′ sequence of the zeta HVR revealing the NcoI sites;

Figure 6 shows the sequence of the interzeta HVR; and

Figure 7 shows the sequences of various repeat types of the 3′ HVR.

The invention further includes the following features:

A polynucleotide in isolated or cloned form having the general formula, read in the 5′ → 3′ sense

H.(J.core.K)$_n$.L, wherein 'core' represents any of the sequences having at least 6 consecutive nucleotides from within a common core region of a plurality of hypervariable regions or human or animal genomic DNA which displays at least 75% concensus; 'core' does not necessarily have the same sequence in each repeating unit and all other symbols are as defined in claim 1 herein, or a polynucleotide of complementary sequence to the above, the polynucleotide being capable of hybridising with fragments of DNA from more than one hypervariable region generated by one or more restriction enzymes which do not cleave within a sequence corresponding to the repeating unit (J.core.K).

Polynucleotides as claimed herein may be prepared by microbiological reproduction of isolated or cloned material, or by direct synthesis.

The polynucleotide probes as claimed herein may be wholly in single-stranded form.

In a method of characterising a test sample of animal or human genomic DNA, a hybridised fragment may be isolated to obtain a secondary probe which is locus-specific for a hypervariable region of the human or animal genome. Said hybridised fragment may be fragment observed by the comparison of individual fragments produced by one or more of the probes in a family or pedigree analysis to be linked to a disease, abnormality or trait whereby said secondary probe is linked with said inherited disease, abnormality or trait. The secondary probe may be obtained from a said fragment observed to be associated with a chromosome or DNA abnormality associated with cancer.

Comparison may be made of patterns of positive fragments obtained using at least two different probes.

The contents of the United Kingdom priority patent application No. 8716367 dated 10th July 1987 are incorporated herein by reference.

## Claims

1. A polynucleotide in insolated or cloned form having the formula, read in the $5' \to 3'$ sense
H.(J.core.K)$_n$.L
wherein:
(a) 'core' represents a sequence having at least 6 consecutive nucleotides, selected from the following sequences:
GXGGGGYACAG
and wherein:
(b) (i) X is A or C or T or G
and Y is A or C or T or G
or nucleotide Y may be absent;
(ii) J and K together represent 0 to 25 additional nucleotides within the repeating unit; and
(iii) H and L each represent 0 or at least 1 additional nucleotide flanking the repeating units;
(iv) the repeating unit (J.core.K) need not be an exact repeat either as to number and kind of nucleotides, provided that the polynucleotide has a recognisably repeated core sequence;
(v) 'core' can also represent a variant core sequence, provided that actual core sequences in all $\underline{n}$ repeating units have on average at least 70% homology with true 'core' sequences as defined above with respect to formula (2),
or a polynucleotide of complementary sequence to the above, the polynucleotide being capable of hybridising with fragments of DNA from more than one hypervariable region generated by one or more restriction enzymes which do not cleave within a sequence corresponding to the repeating unit (J.core.K).

2. A polynucleotide in isolated or cloned form having the general formula, read in the $5' \to 3'$ sense
H.(J.core.K)$_n$.L
wherein H.J.K.L. and 'n' are as defined in claim 1 and:
'core' represents a sequence of from 6 to 16 consecutive nucleotides, read in the same sense, selected from
(1) the common core region of a first animal or human hypervariable region obtained by probing animal or human genomic DNA with a probe containing a $3'$ alpha-globin, or zeta-intron, or interzeta tandem repeat sequence
(2) the common core region of a second animal or human hypervariable region obtained by probing animal or human genomic DNA with a probe containing a tandem repeat sequence comprising (1)
(3) the common core region of a third animal or human hypervariable region obtained by probing animal or human genomic DNA with a probe containing a tandem repeat sequence comprising (2)
each tandem repeat sequence being a repeat of at least 3 units, or a polynucleotide complementary to the aboe, the polynucleotide being capable of hybridising with fragments of DNA from more than one hypervariable region produced by one or more restriction enzymes which do not cleave within a sequence corresponding to the repeating unit (J.core.K).

3. A method of preparing a polynucleotide having polymorphic hypervariable region-length-specific binding characteristics comprising
(i) identifying a natural tandem repeat sequence in DNA which is capable of limiting hybridisation to other polymorphic DNA regions,
(ii) identifying a natural concensus core sequence of the repeat sequence putatively responsible for such binding, and
(iii) isolating or artificially building a perfect or imperfect tandem repeat sequence derived from the natural concensus core sequence having hypervariable region binding properties which exhibits lower genome locus specificity and higher polymorphic fragment acceptance than the natural repeat sequence.

4. A polynucleotide probe comprising, with the inclusion of a labelled or marker component, a polynucleotide comprising at least three tandem repeats (not necessarily exact repeats) of sequences which are homologous with a hypervariable region of the human or animal genome to a degree enabling hybridisation of the probe to a corresponding DNA fragment obtained by fragmenting the same DNA with a restriction endonuclease,
wherein:

(a) the repeats each contain a core which has at least 75% homologous with a consensus core region of similar length present in a plurality of hypervariable regions from different genomic loci;

(b) the core is at least 6 nucleotides long;

(c) the total number of nucleotides within the repeating unit which do not contribute to the core is not more than 25;

(d) the probe is capable of differentiating DNA by reference to more than one polymorphic hypervariable region.

5. A polynucleotide probe characterised in that:

(a) it consists of a labelled polynucleotide, being a polynucleotide as claimed in claim 1 or 2, wherein at least the repeat units are in a single-stranded form and

(b) the probe is capable of differentiating DNA by reference to more than one polynucleotide hypervariable region.

6. A method of preparing a probe useful in genetic origin determination of human or animal DNA-containing samples which comprises introducing a label or marker into a polynucleotide according to claim 1 or 2 or as prepared by the method of claim 3.

7. A method of characterising a test sample of animal or human genomic DNA, by reference to one or more controls, which comprises probing the test sample with a polynucleotide probe comprising, with the inclusion if necessary of a labelled or marker component, at least three tandem repeats, not necessarily exact repeats or sequences which are homologous with a hypervariable region of a genome to a degree enabling hybridisation of the probe therewith, detecting hybridising fragments of DNA, and comparing the hybridised fragments with a said control or controls, wherein:

(a) the sample DNA is fragmented with one or more restriction enzymes which do not cleave to any relevant extent a sequence corresponding to the said tandem repeat;

(b) the repeats each contain a core which is at least 75% homologous with a concensus core region of similar length present in a plurality of hypervariable regions from a different genomic loci;

(c) the core is at least 6 nucleotides long;

(d) the total number of nucleotides within the repeating unit which do not contribute to the core is not more than 25;

(e) the hybridised fragments differentiate the sample DNA by reference to more than one polymorphic hypervariable region.

8. A method, according to claim 7, of establishing the identity or otherwise of the test sample donor with one or more control sample donors, in which the control sample(s) are similarly fragmented and a comparison is made of the hybridised fragments from the respective samples.

9. A method, according to claim 7, of establishing a family connection between the test sample donor and one or more control sample donors, in which the control sample(s) are similarly fragmented and a comparison is made of the hybridised fragments from the respective samples.

10. A method, according to claim 7, wherein DNA samples from horses are compared for the purpose of equine paternity testing, equine pedigree validation, or verification of the identities of specific horses.

FIG.1

FIG.2

FIG.3

EP 0 298 656 A1

FIG.4

EP 0 298 656 A1

GGAGGGGACCTTGGGGAGGGGACAGTGAGGAGGGAACCGTGGAGAGGGGACAGTGAGGAAGGGACAGTGAGGACAGA------------------------

NcoI

GGAGGGGACCATGGGAAGGGGACCGTGGAGTGGGGACAGTGAGGAGGGGACCATAGGGAGGGGACAGTGGGGAGGGGACAGTGAGGAGGGGACCGTGGGG

--------------------------------------------------------------------------------------------

AGGGGACAGTGAGGAGGGGACCGTGGGGAGGAGACAGTGAGGAGGGGACCGTAGGGAGGGGACAGTGAGGAGGGGACCGTGGGGAGGGGACAGTGAGGAG

NcoI

GGGACCGTGGGGAGGGGACAGTGAGGAGGGGACCGTGGGAAGGAGACAGTGAGGAGGGGACCTTGGGGAGGGGACAGTGAGGAGGGGACCATGGGGAGGG

FIG.5

```
                              5'...ACACCCATCAATGGGAG
           CACCAGGACACAGATGGAGGCTAATGTCATGTTGTAG
           ACAGGATGGGTGCTGAGCTGACCACACCCACATTATT
           AGAAAATAACAGCACAGGCTTGGGGTGGAGGCGGGAC
           ACAAGACTAGCCAGAAGGAGAAAGAAAGGTGAAAAGC
           TGTTGGTGCAAGGAAGCTCTTGGTATTTTCAACGGCT
    1   TGGGCACAGGCTGTGAGG–GTGCCTGGGACGGCTTGT
    2   G              CA   AG      CA
    3   G          T        AG      CT
    4   G              CA   AG      CA
    5   G          T        AG      CT
    6   G                   AG      CA
    7   G                    –      C
    8   G          T        AG      C
    9   G          T –      AG      C
   10   G          T        AG      C
   11   G   A      T        AG
   12   G                    –            T
   13   G          T        AG      C     T
   14   G          T        AG      C
   15   G          T        AC      C
   16   G                    –      C      T
   17   G             CA     AG     C
   18   G                    –      C
   19   G                    –      C          A      C
   20   G          T        AG      C
   21   G                    –
   22   G          T        AG      C
   23   G          T        –A      C      T
   24   G          T        AG
   25   G                    –      C
   26   G                    AG
   27   G                    –A     C      T
   28   G          T         G      C
   29   G                    –      CA     T
   30   G             CA     AG     CA
   31   G          T        AA      C
   32   G                    – A    C   C              C
           AGCTACAGGGAGAAAAGACTTGGTGCTGTGGGCCTGC
           CTTGGGGCTGGTGGTACAGCCCTTATCTGCTGCCCTC
           AGGATCTCCCGGCCCCTCTCGTCCAGGCCCCTGCAAC
           CCCATGCCCCAGCCTCTGAGGACCAAAGGCGCCCCTG
           CTTGGGAAGAGGGGGCTCAGGGGAGTCGCCTGACCCG
           GTTCCAAGCCAGGCTGATTTACCGTTGTTAACATCCT
           AGTGCACGCATCCCTCTGCCTCATGCACCCAACTCCA
           AGGCCTGGTACAC...3'
```

*FIG.6*

Nucleotide sequence of the inter-ζ-globin gene HVR. The 1,645-bp segment displayed is from the 4.2-kb *Sac* I D allele and corresponds to the region marked in Fig. 1. To emphasize the tandemly reiterative nature of this region, the full sequence is not illustrated. The sequence on line 1 corresponds to the 5'-most copy of the tandemly reiterated element. Only the differences from copy number 1 in each of the subsequent consecutive 31 elements are indicated. Nucleotides that are absent at a given position are indicated by a dash. The DNA sequence from a position equivalent to elements 27–32 inclusive has been determined from the 4.8-kb *Sac* I D allele and shows 10 base changes

EP 0 298 656 A1

Table   Summary of the 3' HVR composition from sequencing and *Mnl*I mapping

| Repeat type[a] | | Number sequenced | Number from map |
|---|---|---|---|
| *a* | A A C A G C G A C A C G G G G G G | 51 | 133 |
| *b1* | A A C A G C G A C A C G G G A G G | 4 | |
| *b2* | A A C A G C G A C A C G G G G A G G | 2 | 78 |
| *b3* | A A C A G C G A C A C G G G G G A G G | 31 | |
| *b4* | A A C A G C G A C A C G G G G G G A G G | 2 | |
| *c* | A A C A G C G A C A C G G G A G G G A G G | 4 | 12 |

[a] *Mnl*I sites are underlined.

[b] The map cannot distinguish between *b*-type variants.

Table   Representation of the sequence data from the 3' HVR[a]

| | |
|---|---|
| pα3'HVR.64 | AGTCCCACCTGCAGGAAAAGGTGCAGGTAAGG.AATACGGACACGGGAGG.AATACGGACACGGAGGG. |
| | *a a a b3* |
| pMnl.Ii | *b2 a a a a b3 b3 b3 b3 b1 b3 b3 a b3 b3 b3 a a a b3 a b3 b3 b3 b3* |
| pMnl.IIIa | *b3 a a a a a b3 b3 b1 a a c b3 b3* |
| pMnl.IIIc | *b3 a a a a b4 a a a a a a b4 a a* |
| pMnl.IIId | *a a a a a b3 b3 b3 a b3 b3* |
| pMnl.IIIb | *a a a b3 b3 b3 a b1 b3 b3 b2 a a c a a* |
| pα3'HVR.64 | *a a c a b1 a c a a a*  AATACCAACACGGGAGG.  AACAGCAACACGCAGGG. |
| | AATATCGACATGGGTGA.TGCCTGCAAAGCACAGCATCAATCCCAAGTGACTGA. |

[a] The flanking unique sequence and highly diverged repeats are shown in full, the other repeats are represented according to the classification given in Table I.
The *Taq*I site is underlined.

*FIG.7*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 602 948 (LISTER INSTITUTE OF PREVENTIVE MEDICINE) * Whole document * | 1-10 | C 12 Q 1/68 |
| X,P | EP-A-0 238 329 (IMPERIAL CHEMICAL INDUSTRIES PLC) * Page 1, line 3 - page 7, line 57; page 11, example 1; page 15, example 2; page 21, example 3; page 22, line 34 - page 23, line 52; figures 1-12 * | 1-10 | |
| Y | WO-A-8 606 102 (BIOTECHNOLOGY RESEARCH PARTNERS) * Page 4, line 6 - page 5, line 28; page 9, lines 5-32; page 25, table 2; page 30, line 3 - page 33, line 24 * | 1-10 | |
| Y | EP-A-0 164 054 (CETUS CORP.) * Page 4, line 31 - page 6, line 21; page 33, line 2 - page 35, line 29 * | 1-10 | |
| X | NATURE, vol. 314, 7th March 1985, pages 67-73, GB; A.J. JEFFREYS et al.: "Hypervariable 'minisatellite' regions in human DNA" * Page 67, column 2, line 15 - page 71, column 2, line 7; page 72, column 2, lines 7-18 * | 1-10 | TECHNICAL FIELDS SEARCHED (Int. Cl.4) C 12 Q C 12 N |
| X | NATURE, vol. 317, 31st October 1985, pages 818-819, GB; A.J. JEFFREYS et al.: "Positive identification of an immigration test-case using human DNA fingerprints" * Whole article * | 1-10 | |

-/-

The present search report has been drawn up for all claims

| Place of search | Date of completion of the search | Examiner |
|---|---|---|
| THE HAGUE | 09-09-1988 | VAN BOHEMEN C.G. |

European Patent
Office

**EUROPEAN SEARCH REPORT**

Application Number

EP 88 30 5943

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | NATURE, vol. 316, 4th July 1985, pages 76-79; A.J. JEFFREYS et al.: "Individual-specific 'fingerprints' of human DNA" * Whole article * | 1-10 | |
| X | AMERICAN JOURNAL OF HUMAN GENETICS, vol. 39, 1986, pages 11-24, American Society of Human Genetics, US; A.J. JEFFREYS et al.: "DNA "Fingerprints" and segregation analysis of multiple markers in human pedigrees" * Page 11, lines 1-26; page 13, line 11 - page 23, line 15 * | 1-10 | |
| X | JOURNAL OF MEDICAL GENETICS, vol. 23, no. 5, 1986, page 471, US; M.D.A. CRAWFORD et al.: "Minisatellite DNA determination of zygosity in twins discordant for sacral agenesis" * Page 471, whole abstract * | 1-10 | TECHNICAL FIELDS SEARCHED (Int. Cl.4) |

The present search report has been drawn up for all claims

| Place of search | Date of completion of the search | Examiner |
|---|---|---|
| THE HAGUE | 09-09-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                     

& : member of the same patent family, corresponding document

EPO FORM 1503 03.82 (P0401)